# EUROPÄISCHE PATENTSCHRIFT

(11) **EP 1 392 192 B1**
(45) Veröffentlichungstag und Bekanntmachung des Hinweises auf die Patenterteilung: **16.08.2006**
(21) Anmeldenummer: 02732749.3
(22) Anmeldetag: 04.06.2002
(51) Int. Cl.: A61B 18/14

(54) **BIPOLARE KLEMME**
BIPOLAR CLAMP
PINCE BIPOLAIRE

(30) Priorität: 05.06.2001 DE 10127259; 07.02.2002 DE 10205093
(43) Veröffentlichungstag der Anmeldung: 03.03.2004
(73) Patentinhaber: Erbe Elektromedizin GmbH, 72072 Tübingen (DE)
(72) Erfinder: BARTEL, Volker, 72411 Bodelshausen (DE); HILLER, Jürgen, 72581 Dettingen/Erms (DE)
(74) Vertreter: Bohnenberger, Johannes
(86) Internationale Anmeldenummer: PCT/EP2002/006141
(87) Internationale Veröffentlichungsnummer: WO 2002/098313

(56) Entgegenhaltungen:
- WO-A-99/23959
- DE-A- 19 751 108
- DE-U- 9 204 553
- US-A- 4 370 980
- US-A- 5 026 370

## Beschreibung

Die Erfindung betrifft eine bipolare Klemme nach dem Oberbegriff von Patentanspruch 1.

Derartige bipolare Klemmen sind beispielsweise aus den PCT-Anmeldungen WO99/23933 und WO99/23959 bekannt. Sie bestehen im wesentlichen aus zwei Klemmenteilen, die mittels einer isolierenden Schlußschraube oder dergleichen Gelenkverbindung mechanisch gegeneinander verschwenkbar verbunden, aber elektrisch voneinander isoliert sind. An den distalen Enden der Klemmenteile sind Elektrodenteile zum Fassen von Gewebe und zum Durchleiten eines Koagulationsstromes durch das Gewebe vorgesehen. Zum Handhaben der bipolaren Klemme weisen die Klemmenteile an ihren proximalen Enden Griffeinrichtungen auf. Der Koagulationsstrom wird mittels Stromzuführungseinrichtungen am proximalen Ende mindestens eines Klemmenteils zugeführt. Um die bipolare Klemme in einer Schließposition verriegeln zu können, sind zwischen der Gelenkverbindung und den proximalen Enden der Klemmenteile Rasteinrichtungen vorhanden. In der Schließposition ist so ein sicheres Halten von Gewebe mit der Klemme gewährleistet.

Die aus der WO99/23933 bekannte bipolare Klemme weist jedoch zwei Nachteile auf.

Eine Stromzuführungseinrichtung ist nur an einem Klemmenteil, genauer gesagt an der am proximalen Ende des Klemmenteils angebrachten Griffeinrichtung vorgesehen. Die Stromzuführungseinrichtung wird durch zwei Drähte gebildet, die etwa bis zur Gelenkverbindung der beiden Klemmenteile der Klemme gemeinsam geführt sind. Ab der Gelenkverbindung werden die Drähte getrennt zu den Elektroden geführt, mit denen sie elektrisch verbunden sind. Zwar läßt sich die Klemme dadurch einfacher handhaben, jedoch ist die Führung der Drähte insbesondere im Bereich der Gelenkverbindung der Klemmenteile vor allem bei häufiger Betätigung der Klemme verschleißanfällig; es kann ein Bruch in den elektrischen Leitungen auftreten. Aufgrund der hohen mechanischen Belastung ist dies bei dieser Art von Klemme sehr wahrscheinlich.

Zudem ist es möglich, dass bereits vor einer Verriegelung der Klemmenteile in der Schließposition ein Koagulationsstrom fließt, da der Zeitpunkt des Stromflusses vom Operateur bestimmt wird. Dieser muss mit der Klemme daher im wesentlichen drei Handgriffe zum Koagulieren von Gewebe ausführen: Greifen von Gewebe, Verriegeln der Klemmenteile und Aktivieren des Stromflusses. Hierdurch gestaltet sich die Handhabung aufwendig. Vor allem kann es leicht vorkommen, dass der Operateur versehentlich vor dem Verriegeln den Stromfluss aktiviert, wodurch unbeabsichtigt eine Koagulation ausgelöst werden kann.

Aufgabe der vorliegenden Erfindung ist es daher, die bekannte bipolare Klemme derart weiter zu bilden, daß sie vor allem sicherer und ferner einfacher gehandhabt werden kann.

Diese Aufgabe wird durch eine bipolare Klemme mit den Merkmalen nach Patentanspruch 1 gelöst. Bevorzugte Ausgestaltungen dieser bipolaren Klemme ergeben sich aus den abhängigen Patentansprüchen.

Der Erfindung liegt die Idee zugrunde, die Stromzuführungseinrichtungen derart anzuordnen und die Rasteinrichtungen derart auszubilden, dass nur in der Schließposition ein Stromfluss von den Stromzuführungseinrichtungen zu den Elektrodenteilen ermöglicht ist. Hierdurch wird eine besonders sichere bipolare Klemme geschaffen, da ein Stromfluss erst möglich ist, wenn Gewebe mit der Klemme gefasst ist und sich die Klemme in der Schließposition befindet. Mit anderen Worten kann eine Koagulation von Gewebe erst stattfinden, wenn das Gewebe mit der Klemme sicher gefasst und zwischen die Klemmenteile eingeklemmt ist. Ein zu frühes Zuführen von Koagulationsstrom an mit der Klemme zu greifendes Gewebe wird somit zuverlässig verhindert. Außerdem wird die Handhabung vereinfacht, da im Prinzip der Stromfluss durch das Verriegeln der Klemmenteile gesteuert wird. D.h. ein Operateur braucht nur noch das zu koagulierende Gewebe zu fassen und die Klemmenteile zu verriegeln, um eine Koagulation zu bewirken.

Gemäß der Erfindung umfasst die bipolare Klemme zwei Klemmenteile, die mittels einer isolierenden Schlußschraube oder dergleichen Gelenkverbindung mechanisch gegeneinander verschwenkbar verbunden, aber elektrisch voneinander isoliert sind. An distalen Enden der Klemmenteile sind Elektrodenteile zum Fassen von Gewebe und zum Durchleiten eines Koagulationsstromes durch das Gewebe vorgesehen. Die proximalen Enden der Klemmenteile umfassen Griffeinrichtungen zum einfachen Handhaben der bipolaren Klemme. Ferner sind Stromzuführungseinrichtungen vorgesehen, über die der Koagulationsstrom zuführbar ist. Zwischen der Gelenkverbindung und den proximalen Enden sind zum Verriegeln der Klemmenteile aneinander in einer Schließposition Rasteinrichtungen vorgesehen. Die Stromzuführungseinrichtungen sind derart angeordnet und die Rasteinrichtungen derart ausgebildet, dass nur in der Schließposition ein Stromfluss von den Stromzuführungseinrichtungen zu den Elektrodenteilen ermöglicht ist.

Die Stromzuführungseinrichtungen sind vorzugsweise, am proximalen Ende eines Klemmenteils angeordnet und die Rasteinrichtungen bilden einen Leitungsabschnitt der Stromzuführungseinrichtungen. Dadurch können die Stromzuführungseinrichtungen weit vor der Gelenkverbindung im proximalen Bereich eines der Klemmenteile getrennt geführt und von einer Betätigung der Klemme im wesentlichen nicht beeinflußt werden. Hierbei eignen sich die insbesondere metallisch blanken Rasteinrichtungen als Leitungsabschnitt einer der Stromzuführungseinrichtungen. Vor allem kann eine Aufspaltung - wie bei der aus der W099/23933 bekannten bipolaren Klemme - der bis zur Gelenkverbindung gemeinsam und ab dort getrennt geführten Drähte als Stromzuführungseinrichtungen vermieden werden. Eine mechanische Belastung der Drähte im Bereich der Gelenkverbindung beim Öffnen und Schließen der Klemme findet nicht mehr statt; die Klemme ist weniger verschleißanfällig und weniger ausfallgefährdet.

Die Stromzuführungseinrichtungen können elektrische Leitungen und zumindest abschnittsweise außerhalb und/oder innerhalb der Klemmenteile geführt sein. Bei einer Führung außerhalb der Klemmenteile sollten die elektrischen Leitungen möglichst elektrisch isoliert und an dem oder den Klemmenteil(en) fixiert sein. Als Fixierung kommt vorzugsweise eine Kleb-, eine Klemmverbindung oder eine Fixierung mittels spezieller, an einem Klemmenteil vorgesehener Führungseinrichtungen in Betracht. Bei einer Führung einer elektrischen Leitung innerhalb eines Klemmenteils ist es erforderlich, daß das Klemmenteil entweder hohl ist oder einen Führungskanal, beispielsweise in Form einer Bohrung oder einer Nut (zur Aufnahme einer Leitung) aufweißt.

Für den Fall von Führungseinrichtungen, durch die elektrische Leitungen geführt sind, bestehen die Klemmenteile bevorzugt zumindest teilweise aus einem elektrisch isolierenden Material. In diesem Fall können auch blanke Drähte als Stromzuführungseinrichtungen verwendet werden. Hierbei sollten die Rasteinrichtungen ein elektrisch leitfähiges Rastteil und ein Trägerteil umfassen. Das Rastteil ist derart auf dem Trägerteil befestigt, daß zwischen den beiden Teilen ein Zwischenraum zur Aufnahme eines elektrischen Leitungsabschnitts besteht, der mit dem Rastteil elektrisch verbunden ist, insbesondere mittels einer Lötverbindung.

Vorzugsweise sind an dem die Stromzuführungseinrichtungen aufweisenden Klemmenteil zwei elektrische Leitungen geführt. Eine der Leitungen ist an das Elektrodenteil im distalen Ende des die Stromzuführungseinrichtung aufweisenden Klemmenteils geführt. Die andere Leitung ist an das Rastteil der Rasteinrichtung geführt und mit diesem elektrisch verbunden. Das andere Klemmenteil besteht vorzugsweise integral mit dem am distalen Ende angebrachten Elektrodenteil vollständig aus elektrisch leitfähigem Material. Ein elektrischer Stromfluß wird somit über die Rasteinrichtungen geführt, genauer gesagt über eine elektrische Leitung bis zum Rastteil der ersten Rasteinrichtung und über das Rastteil der zweiten Rasteinrichtung durch das Klemmenteil bis zur Elektrode. Eine derartige bipolare Klemme kommt mit einem Minimum an elektrischen Leitungen aus, wodurch sie konstruktiv relativ einfach aufgebaut ist.

In einer weiteren bevorzugten Ausführungsform der erfindungsgemäßen Klemme sind die Stromzuführungseinrichtungen an einer der Rasteinrichtungen angeordnet; die Klemmenteile bestehen aus einem leitfähigen Material, insbesondere aus einem Metall, und sind außen elektrisch isoliert; ferner bilden die Rasteinrichtungen einen Leitungsabschnitt der Stromzuführungseinrichtungen. Hierdurch wird ein Instrument geschaffen, das insbesondere bei der metallenen Ausführung der Klemmenteile mechanisch hoch belastbar ist und den wesentlichen Vorteil der Erfindung, nämlich die sichere Handhabung aufweist. Beispielsweise kann ein zweipoliger elektrischer Stecker als Stromzuführungseinrichtung direkt an einer der Rasteinrichtungen angeordnet sein. Aufgrund der aus einem leitfähigen Material bestehenden Klemmenteile dienen diese als elektrische Stromleitungen, über die Koagulationsstrom zu den Elektroden geleitet werden kann. Die äussere elektrische Isolierung der Klemmenteile verhindert eine Schädigung von Gewebe, das mit den Klemmenteilen in Berührung kommt. Um die Koagulationsfunktion erst in der Schließposition der Klemme zu ermöglichen, bilden die Rasteinrichtungen einen Leitungsabschnitt der Stromzuführungseinrichtungen. Diese Ausführungsform besitzt nur wenige Teile und ist daher einerseits kostengünstig und andererseits wenig verschleißanfällig.

Um die Klemmenteile sicher voneinander elektrisch zu isolieren, umfaßt die Gelenkverbindung einen Zapfen als Drehachse, der in einem der Klemmenteile in einer isolierenden Buchse, insbesondere aus Keramik, gelagert ist. Die Lagerung in einer Keramikbuchse hat den Vorteil, daß die Gelenkverbindung besonders verschleißarm ist. Ferner weist Keramik besonders gute elektrische Isolationseigenschaften auf. Es kann aber auch eine isolierende Buchse aus beispielsweise Kunststoff verwendet werden, die eine kostengünstige Ausführung der bipolaren Klemme ermöglicht. Die Buchse sollte hierbei derart isolierend sein, daß kein wesentlicher Stromfluß zwischen den beiden Klemmenteilen über die Gelenkverbindung auftreten kann. In der einfachsten Ausführung kann die Buchse eine isolierende Beschichtung aufweisen, die einen sehr hohen Übergangswiderstand für einen Strom darstellt.

Im folgenden wird nun ein Ausführungsbeispiel der erfindungegemäßen bipolaren Klemme anhand der Zeichnungen erläutert. Diese zeigen in
- Fig. 1: ein Ausführungsbeispiel der erfindungsgemäßen bipolaren Klemme
- Fig. 2: die in Fig. 1 abgebildete bipolare Klemme mit gestrichelt dargestellten elektrischen Leitungen;
- Fig. 3: eine isolierende Gelenkverbindung der in Fig. 1 dargestellten bipolaren Klemme geschnitten in Seitenansicht und
- Fig. 4: eine der Rasteinrichtungen der bipolaren Klemme aus Fig. 2 im Längsschnitt.

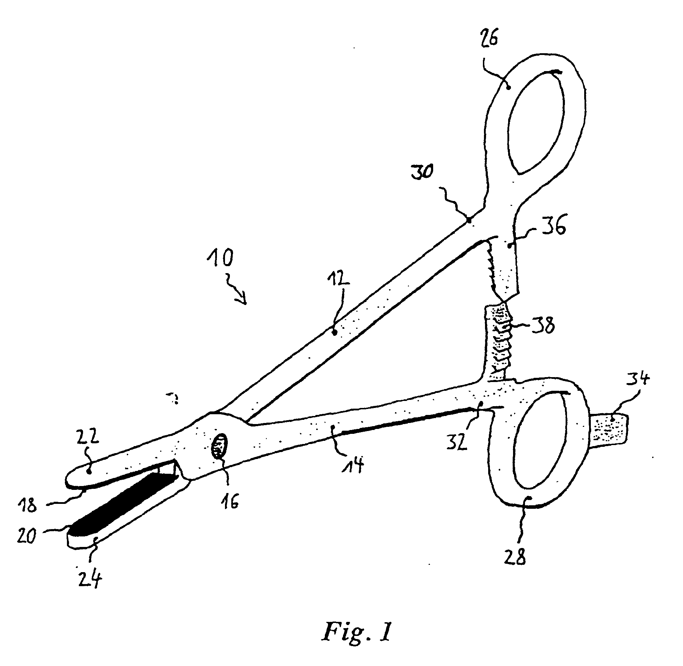

Die in Fig. 1 in perspektivischer Ansicht gezeigte bipolare Klemme 10 umfaßt zwei Klemmenteile 12 und 14. Jedes der Klemmenteile 12 und 14 ist jeweils an seinem proximalen Ende 30 bzw. 32 mit einer Griffeinrichtung 26 bzw. 28 versehen. Die Griffeinrichtungen 26 bzw. 28 sind als Ösen ausgebildet, ähnlich wie bei einer Schere, und daher von einem Operateur einfach handhabbar. Die Klemmenteile 12 und 14 sind durch eine Gelenkverbindung 16 mechanisch gegeneinander verschwenkbar verbunden. Die Gelenkverbindung 16 ist nahe den distalen Enden 22 und 24 der Klemmenteile 12 bzw. 14 vorgesehen. An den distalen Enden 22 und 24 der Klemmenteile 12 bzw. 14 sind elektrisch leitfähige Elektrodenteile 20 bzw. 18 angebracht, die einerseits zum Fassen von Gewebe und andererseits zum Durchleiten eines Koagulationsstromes durch das Gewebe dienen. Hierzu sind sie vorzugsweise aus Metall ausgebildet. Am Griffteil 28 des Klemmenteils 14 sind Stromzuführungseinrichtungen 34 vorgesehen. Diese dienen zum Anschließen an einen (nicht dargestellten) HF-Generator, der einen hochfrequenten elektrischen Koagulationsstrom erzeugt, der über die Stromzuführungseinrichtungen den Elektrodenteilen 18 und 20 zugeführt wird.

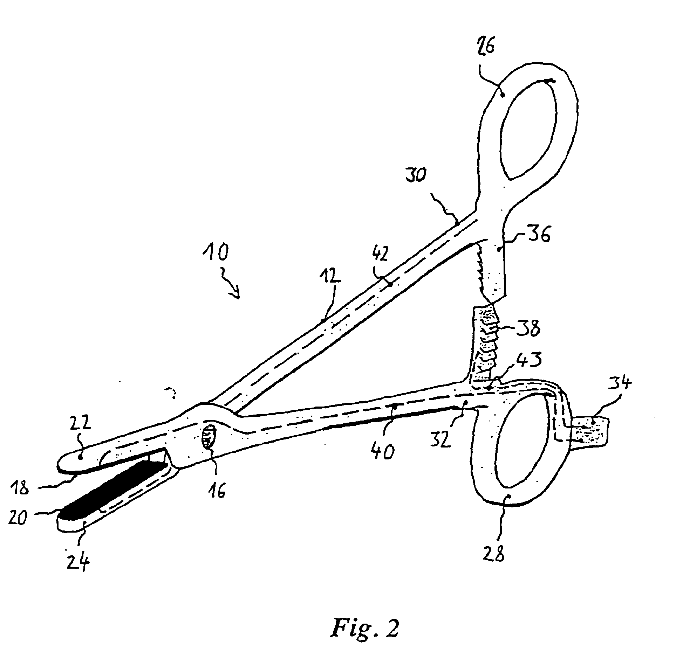

Fig. 2 verdeutlicht mittels gestrichelter Linien in der in Fig. 1 dargestellten Klemme 10 die Führung der Stromzuführungseinrichtungen. Die Stromzuführungseinrichtungen sind als elektrisch isolierte Leitungen 40, 42 und 43 ausgebildet, die innerhalb der Klemmenteile 12 und 14 geführt sind. Die elektrische Leitung 43 ist vom Anschlußpunkt der Stromzuführungseinrichtungen 34, der sich an der Griffeinrichtung 28 des Klemmenteils 14 befindet, zur ersten Rasteinrichtung 38 am proximalen Ende 32 des Klemmenteils 14 geführt. Eine weitere elektrische Leitung 42 ist an einem Ende mit der zweiten Rasteinrichtung 36 am proximalen Ende 30 und am anderen Ende mit dem Elektrodenteil 20 am distalen Ende des Klemmenteils 12 elektrisch verbunden. Die elektrische Leitung 42 kann hierbei entweder außen am Klemmenteil 12 oder auch innerhalb des Klemmenteils 12 geführt sein. Wesentlich ist hierbei, daß die elektrische Leitung 42 nicht von der Gelenkverbindung 16 beeinflußt, d. h. beim Bewegen der beiden Klemmenteile 12 und 14 gebogen wird. Damit ist beispielsweise die Gefahr eines Leitungsbruchs aufgrund häufiger Benutzung der Klemme geringer als bei der aus der WO99/23933 bekannten bipolaren Klemme. Eine dritte Leitung 40 führt vom Anschlußpunkt der Stromzuführungseinrichtungen 34 direkt zum Elektrodenteil 18 am distalen Ende 22 des Klemmenteils 14. Auch diese Leitung 40 wird beim Benutzen der bipolaren Klemme 10 nicht gebogen. In Fig. 2 ist die Leitung 40 von oberhalb über die Gelenkverbindung 16 geführt. Sie kann selbstverständlich auch anders geführt sein, beispielsweise unterhalb der Gelenkverbindung 16.

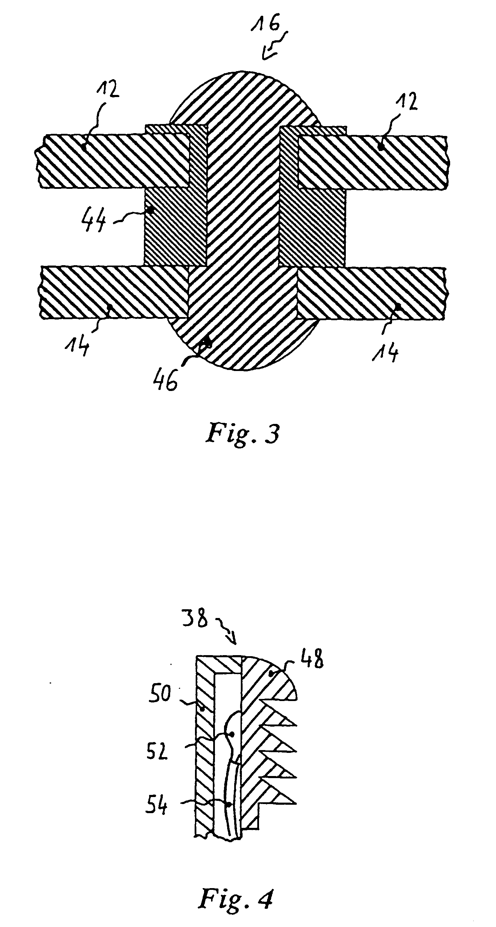

Fig. 3 zeigt im Detail den Aufbau der Gelenkverbindung 16. Diese umfaßt einen Zapfen 46, der ähnlich einer Niet die Klemmenteile 12 und 14 miteinander gelenkig verbindet. Der Zapfen ist mit einem Ende in einer Buchse 44 gelagert, die in einer dafür vorgesehen Öffnung des Klemmenteils 12 eingeklemmt ist. Die Buchse 44 ist elektrisch isolierend, insbesondere aus Keramik. Sie kann auch aus einem elektrisch sehr schwach leitenden Material, d. h. einem Material mit einem sehr hohen elektrischen Widerstand bestehen, so daß ein Stromfluß über die Gelenkverbindung 16 vom Klemmenteil 14 zum Klemmenteil 12 oder umgekehrt im Vergleich zum Koagulationsstromfluß vernachlässigbar gering ist.

Schließlich zeigt die Fig. 4 den Aufbau der Rasteinrichtung 38 im Schnitt. Die Rasteinrichtung 38 umfaßt ein Rastteil 48, das auf einem Trägerteil 50 angebracht ist, welches wiederum einstückig mit dem Klemmenteil 14 ausgebildet ist. Das Trägerteil 50 besteht aus einem isolierenden Material. Das aus einem leitenden Material bestehende Rastteil 48 ist mittels einer Lötverbindung 52 mit einem Leitungsabschnitt 54 der elektrischen Leitung 43 elektrisch verbunden. Es kann auf dem Trägerteil 50 insbesondere klebend oder klemmend befestigt sein.

### Bezugszeichen

- 10: bipolare Klemme
- 12: Klemmenteil
- 14: Klemmenteil
- 16: isolierende Gelenkverbindung
- 18: Elektrodenteil
- 20: Elektrodenteil
- 22: distales Ende eines Klemmenteils
- 24: distales Ende eines Klemmenteils
- 26: Griffeinrichtung
- 28: Griffeinrichtung
- 30: proximales Ende eines Klemmenteils
- 32: proximales Ende eines Klemmenteils
- 34: Stromzuführungseinrichtungen
- 36: Rasteinrichtung
- 38: Rasteinrichtung
- 40: elektrische Leitung
- 42: elektrische Leitung
- 43: elektrische Leitung
- 44: isolierende Buchse
- 46: Zapfen
- 48: Rastteil
- 50: Trägerteil
- 52: Lötverbindung
- 54: Leitungsabschnitt

## Patentansprüche

1. Bipolare Klemme (10), umfassend
- zwei Klemmenteile (12, 14), die mittels einer isolierenden Schlußschraube oder dergleichen Gelenkverbindungen (16) mechanisch gegeneinander verschwenkbar verbunden, aber elektrisch voneinander isoliert sind,
- Elektrodenteile (18, 20) an distalen Enden (22, 24) der Klemmenteile (12, 14) zum Fassen von Gewebe und zum Durchleiten eines Koagulationsstromes durch das Gewebe,
- Griffeinrichtungen (26, 28) an proximalen Enden (30, 32) der Klemmenteile (12, 14),
- Stromzuführungseinrichtungen (34, 40, 42)
- Rasteinrichtungen (36, 38) zwischen der Gelenkverbindung (16) und den proximalen Enden (32, 34) zum Verriegeln der Klemmenteile (12, 14) aneinander in einer Schließposition,
**dadurch gekennzeichnet, daß**
die Stromzuführungseinrichtungen (34, 40, 42) derart angeordnet und die Rasteinrichtungen (36, 38) derart einen Leitungsabschnitt der Stromzuführungseinrichtungen (34, 40, 42, 43) bilden, dass nur in der Schließposition ein Stromfluss von den Stromzuführungseinrichtungen (34, 40, 42) zu den Elektrodenteilen (18, 20) ermöglicht ist.

2. Bipolare Klemme nach Anspruch 1,
**dadurch gekennzeichnet, daß**
die Stromzuführungseinrichtungen (34, 40, 42) am proximalen Ende eines Klemmenteils (32) angeordnet sind.

3. Bipolare Klemme nach Anspruch 1 oder 2,
**dadurch gekennzeichnet, daß**
die Stromzuführungseinrichtungen (34) elektrische Leitungen (40, 42, 43) sind, die zumindest abschnittsweise außerhalb und/oder innerhalb der Klemmenteile (12, 14) geführt sind.

4. Bipolare Klemme nach einem der vorhergehenden Ansprüche, insbesondere nach Anspruch 3,
**dadurch gekennzeichnet , daß**
die Klemmenteile (12, 14) zumindest teilweise aus einem elektrisch isolierenden Material bestehen und die elektrischen Leitungen mittels Führungseinrichtungen, insbesondere Nuten, Bohrungen oder Kanälen an bzw. in den Klemmenteilen (12, 14) geführt sind.

5. Bipolare Klemme nach einem der vorhergehenden Ansprüche, insbesondere nach Anspruch 4,
**dadurch gekennzeichnet, daß**
mindestens eine Rasteinrichtung (38) ein elektrisch leitfähiges Rastteil (48) und ein Trägerteil (50) umfasst, wobei das Rastteil (48) auf dem Trägerteil (50) derart befestigt ist, daß zwischen Rast- und Trägerteil (48, 50) ein Zwischenraum zur Aufnahme eines elektrischen Leitungsabschnitts (54) besteht, der mit dem Rastteil (48) elektrisch verbunden ist, insbesondere mittels einer Lötverbindung.

6. Bipolare Klemme nach einem der vorhergehenden Ansprüche, insbesondere nach Anspruch 5,
**dadurch gekennzeichnet, daß**
in dem die Stromzuführungseinrichtungen aufweisenden Klemmenteil (14) zwei elektrische Leitungen (40, 43) geführt sind, von denen eine.an das Elektrodenteil (18) am distalen Ende (22) des Klemmenteils (14) und die andere an das Rastteil (48) der Rasteinrichtung (38) geführt und jeweils damit elektrisch verbunden sind, wobei das andere Klemmenteil (12) aus elektrisch leitfähigem Material besteht.

7. Bipolare Klemme nach einem der vorhergehenden Ansprüche, insbesondere nach Anspruch 1,
**dadurch gekennzeichnet, daß**
die Stromzuführungseinrichtungen (34, 40, 42) an einer der Rasteinrichtungen (36) angeordnet sind, die Klemmenteile (12, 14) aus einem leitfähigen Material, insbesondere aus einem Metall, bestehen und außen elektrisch isoliert sind und die Rasteinrichtungen (36, 38) einen Leitungsabschnitt der Stromzuführungseinrichtungen (34, 40, 42, 43) bilden.

8. Bipolare Klemme nach einem der vorhergehenden Ansprüche,
**dadurch gekennzeichnet, daß**
die elektrisch isolierende Gelenkverbindung (16) einen Zapfen (46) als Drehachse umfaßt, der in einem der Klemmenteile (12) in einer isolierenden Buchse (44), insbesondere aus Keramik, gelagert ist.

## Claims

1. Bipolar clamp (10) comprising
- two clamp members (12, 14), which are joined together mechanically by means of an insulating locking screw or similar pivot joints (16) about which they can rotate with respect to one another, but are electrically insulated from one another,
- electrode components (18, 20) at distal ends (22, 24) of the clamp members (12, 14) for grasping tissue and conducting a coagulation current through the tissue,
- handle devices (26, 28) at proximal ends (30, 32) of the clamp members (12, 14),
- current-supply means (34, 40, 42),
- latch elements (36, 38) between the pivot joint (16) and the proximal ends (32, 34) to lock the clamp members (12, 14) to one another in a closed position,
**characterized in that**
the current-supply means (34, 40, 42) are disposed, and the latch elements (36, 38) form a conducting section of the current-supply means (34, 40, 42), in such a way that current flow from the current-supply means (34, 40, 42) to the electrode components (18, 20) is possible only in the closed position.

2. Bipolar clamp according to Claim 1,
**characterized in that**
the current-supply means (34, 40, 42) are disposed at the proximal end of a clamp member (32).

3. Bipolar clamp according to Claim 1 or 2,
**characterized in that**
the current-supply means (34) are electrical leads (40, 42, 43) that are at least partially guided along the exterior of and/or within the clamp members (12, 14).

4. Bipolar clamp according to one of the preceding claims, in particular Claim 3,
**characterized in that**
the clamp members (12, 14) consist at least partially of an electrically insulating material and the electrical leads are guided by guide devices, in particular grooves, bores or channels on or in the clamp members (12, 14).

5. Bipolar clamp according to one of the preceding claims, in particular Claim 4,
**characterized in that**
at least one latch element (38) comprises an electrically conductive locking part (48) and a carrier part (50) , the locking part (48) being fixed to the carrier part (50) in such a way that between the locking and carrier parts (48, 50) there is a gap to accommodate an electrical lead section (54) that is electrically connected to the locking part (48), in particular by a solder connection.

6. Bipolar clamp according to one of the preceding claims, in particular Claim 5,
**characterized in that**
within the clamp member (14) containing the current-supply means two electrical leads (40, 43) are guided, of which one runs to the electrode component (18) at the distal end (22) of the clamp member (14) and the other runs to the locking part (48) of the latch element (38) and each is electrically connected therewith, while the other clamp member (12) is made of electrically conductive material.

7. Bipolar clamp according to one of the preceding claims, in particular Claim 1,
**characterized in that**
the current-supply means (34, 40, 42) are disposed at one of the latch elements (36), the clamp members (12, 14) are made of a conductive material, in particular of metal, and externally are electrically insulated, and the latch elements (36, 38) form a conducting section of the current-supply means (34, 40, 42, 43).

8. Bipolar clamp according to one of the preceding claims,
**characterized in that**
the electrically insulating pivot joint (16) comprises as axis of rotation a pin (46), which is seated in one of the clamp members (12) within an insulating socket (44), in particular made of ceramic.

## Revendications

1. Pince bipolaire (10) comprenant
- deux éléments de pince (12, 14), qui au moyen d'une vis de terminaison isolante ou de liaisons articulées similaires (16) sont reliés de façon mécaniquement pivotante l'une contre l'autre, mais en étant électriquement isolés l'un par rapport à l'autre,
- des éléments à électrodes (18, 20) sur des extrémités distales (22, 24) des éléments de pince (12, 14) pour la préhension de tissus et pour le passage d'un courant de coagulation à travers les tissus,
- des systèmes de manches (26, 28) sur des extrémités proximales (30, 32) des éléments de pince (12, 14),
- des systèmes d'alimentation de courant (34, 40, 42)
- des systèmes d'enclenchement (36, 38) entre la liaison articulée (16) et les extrémités proximales (32, 34) pour verrouiller les éléments de pince (12, 14) l'un contre l'autre dans une position de fermeture
**caractérisée en ce que**
les systèmes d'alimentation de courant (38, 40, 42) sont disposés de façon telle et les systèmes d'enclenchement (36, 38) forment un tronçon de ligne des systèmes d'alimentation de courant (34, 40, 42, 43) de façon telle qu'une circulation de courant des systèmes d'alimentation de courant (34, 40, 42) vers les éléments à électrodes (18, 20) n'est possible qu'en position de fermeture.

2. Pince bipolaire selon la revendication 1,
**caractérisée en ce que** les systèmes d'alimentation de courant (34, 40, 42) sont disposés sur l'extrémité proximale d'un élément de pince (32).

3. Pince bipolaire selon la revendication 1 ou 2,
**caractérisée en ce que** les systèmes d'alimentation de courant (34) sont des lignes électriques (40, 42, 43), qui au moins par tronçons sont tirées à l'extérieur et/ou à l'intérieur des éléments de pince (12, 14).

4. Pince bipolaire selon l'une quelconque des revendications précédentes, notamment selon la revendication 3,
**caractérisée en ce que** les éléments de pince (12, 14) sont au moins partiellement en un matériau isolant et les lignes électriques sont tirées au moyens de systèmes de guidage, notamment de rainures, de perçages ou de canaux sur ou dans les éléments de pince (12, 14).

5. Pince bipolaire selon l'une quelconque des revendications précédentes, notamment selon la revendication 4,
**caractérisée en ce qu'**au moins un système d'enclenchement (38) comprend un élément d'enclenchement (48) conducteur d'électricité et un élément support (50), l'élément d'enclenchement (48) étant fixé sur l'élément support (50) de façon à ménager entre l'élément d'enclenchement et l'élément support (48, 50) un interstice pour loger un tronçon de ligne électrique (54) qui est électriquement relié à l'élément d'enclenchement (48), notamment par liaison soudée.

6. Pince bipolaire selon l'une quelconque des revendications précédentes, notamment selon la revendication 5,
**caractérisée en ce que** deux lignes électriques (40, 43) dont l'une est tirée vers l'élément à électrodes (18) sur l'extrémité distale (22) de l'élément de pince (14) et l'autre est tirée sur l'élément d'enclenchement (48) du système d'enclenchement (38), tout en étant respectivement reliées électriquement avec ces derniers sont tirées dans l'élément de pince (14) comportant les systèmes d'alimentation de courant, l'autre élément de pince (12) étant en matériau conducteur d'électricité.

7. Pince bipolaire selon l'une quelconque des revendications précédentes, notamment selon la revendication 1,
**caractérisée en ce que** les systèmes d'alimentation de courant (34, 40, 42) sont disposés sur l'un des systèmes d'enclenchement (36), les éléments de pince (12, 14) sont en un matériau conducteur, notamment en un métal et sont électriquement isolés sur l'extérieur et les systèmes d'enclenchement (36, 38) forment un tronçon de ligne des systèmes d'alimentation de courant (34, 40, 42, 43).

8. Pince bipolaire selon l'une quelconque des revendications précédentes,
**caractérisée en ce que** la liaison articulée électriquement isolée (16) comprend un tenon (46) en tant qu'axe que rotation, qui est logé dans l'un des éléments de pince (12), dans une douille isolante (44), notamment en céramique.
